# EUROPÄISCHE PATENTANMELDUNG

(11) **EP 1 127 612 A2**
(43) Veröffentlichungstag der Anmeldung: **29.08.2001**
(21) Anmeldenummer: 01103906.2
(22) Anmeldetag: 17.02.2001
(51) Int. Cl.: B01J 19/12

(54) **Solarer Photoreaktor**

(30) Priorität: 25.02.2000 DE 10009060
(71) Anmelder: DLR Deutsches Zentrum für Luft- und Raumfahrt e.V., 53175 Bonn (DE)
(72) Erfinder: Funken, Karl-Heinz, Dr., 53225 Bonn (DE); Sattler, Christian, Dr., 53111 Bonn (DE); Ortner, Jürgen, Dr., 51065 Köln (DE); de Oliveira, Lamark, 51147 Köln (DE)
(74) Vertreter: Jönsson, Hans-Peter, Dr.

(57) **Zusammenfassung**

Die Erfindung betrifft eine Vorrichtung und ein Verfahren zur Einbringung solarer Strahlungsenergie in einen Photoreaktor. Dieser Reaktor kann zur Durchführung photochemischer oder photobiologischer Reaktionen - Synthesen, Reinigungs-, Desinfektions- und Behandlungsverfahren - eingesetzt werden.

Die Vorrichtung zur Durchführung solarer photochemischer oder photobiologischer Reaktionen ist dadurch gekennzeichnet, dass der Photoreaktor aus einem oder mehreren transparenten äußeren Rohren besteht, die von außen durch Licht bestrahlt werden, wobei das Reaktionsmedium durch die Rohre geleitet und dabei der Strahlung ausgesetzt wird, wobei insbesondere konzentrisch in dem Inneren der äußeren Rohre ein oder mehrere leere transparente Rohre mit kleinerem Durchmesser angeordnet sind und das Reaktionsmedium durch den zwischen einem äußeren und einem inneren Rohr entstehenden Spalt oder im Falle mehrerer innerer Rohre durch die zwischen ihnen entstehenden Spalten geleitet wird.

## Beschreibung

Die Erfindung betrifft eine Vorrichtung und ein Verfahren zur Einbringung solarer Strahlungsenergie in einen Photoreaktor. Dieser Reaktor kann zur Durchführung photochemischer oder photobiologischer Reaktionen - Synthesen, Reinigungs-, Desinfektions- und Behandlungsverfahren - eingesetzt werden.

Zur Durchführung photochemischer oder photobiologischer Reaktionen muss elektromagnetische Strahlung bereitgestellt werden. Der wirtschaftliche Erfolg eines photochemischen oder photobiologischen Verfahrens hängt in erheblichem Maße von den Kosten der Strahlungsbereitstellung ab.

Zu den im industriellen Maßstab verwirklichten photochemischen Prozessen mit dem Ziel der Herstellung von Chemikalien zählen beispielsweise Photohalogenierungen, Photosulfochlorierungen, Photosulfoxidationen, Photonitrosierungen, Photoisomerisierungen, Photohydrodimerisierungen, Photodesulfonierungen, Photosulfonylierungen und Photooxygenierungen. Es sind aber auch weitere, noch nicht im industriellen Maßstab durchgeführte Reaktionstypen bekannt, die unter der Einwirkung von elektromagnetischer Strahlung mit großer Selektivität zu veredelten Produkten führen.

Auch durch photobiologische Umsetzungen kann man hochveredelte Produkte gewinnen. Applikationsgebiete für die Produkte, die aus phototrophen Cyanobakterien, Algen und Mikroalgen gewonnen werden, bieten sich in der Medizin, der Pharmazie, der Kosmetik, der Landwirtschaft, der Ernährung sowie im Bereich der Umwelttechnik. Es lassen sich beispielsweise Farbstoffe und färbende Lebensmittel (beispielsweise Phycocyanine, Phycobiliproteine und Carotinoide), mehrfach ungesättigte Fettsäuren (insbesondere Arachidonsäure, Eicosapentaen- und Docosahexaensäure), Antioxidantien (beispielsweise Tocopherol), Proteine und Polysaccharide herstellen. Auch pharmakologisch aktive Substanzen (Bacterizide und Fungizide) wurden in verschiedenen Mikroalgen identifiziert.

Im Gebiet der Umwelttechnik werden photochemische und photobiologische Verfahren entwickelt, um durch Schadstoffe und / oder Mikroorganismen kontaminierte Wässer oder Gasströme zu detoxifizieren und / oder zu entkeimen. Gemeinsam ist bei diesen Verfahren, dass unter photonischer Anregung Singulett-Sauerstoff, Hydroxyl- oder andere Sauerstoff-haltige Radikale gebildet werden, die dann die abzubauenden Schadstoffe bzw. die zu inaktivierenden Mikroorganismen angreifen. So ist die Bildung angeregter Sauerstoff-Spezies möglich und bekannt durch Energietransfer von einem elektronisch angeregten Donor, durch Anregung eines Halbleiter-Materials, wie beispielsweise Titandioxid, oder durch Photo-Fenton-Reagenzien.

Zur Durchführung der photochemischen oder photobiologischen Umsetzungen stehen verschiedene Strahlungs-, insbesondere Lichtquellen zur Verfügung, so beispielsweise Gasentladungslampen, Glühlampen, fluoreszierende Lampen oder Röhren, Excimer-Strahler sowie Laser. Jede dieser Strahlungsquellen besitzt charakteristische Eigenschaften bezüglich der Art des emittierten Spektrums und der Leuchtstärke. Laser können zwar intensive Strahlung bei einer gewünschten Wellenlänge zur Verfügung stellen, aber die Installation und der Betrieb von Lasern ist mit so hohen Kosten verbunden, dass ihr Einsatz aus wirtschaftlichen Gründen nur in sehr speziellen Fällen zu rechtfertigen ist.

Anstelle elektrisch betriebener Lichtquellen kann auch die Sonnenstrahlung zur Durchführung photochemischer und photobiologischer Umsetzungen genutzt werden. Übersichten zum Stand der solaren photochemischen und photokatalytischen Reaktionstechnik wurden bereits publiziert. Hierin wurden auch die wichtigsten für eine Kommerzialisierung zu lösenden Probleme aufgezeigt (K.-H. Funken, D.M. Blake, M. Romero, I. Spiewak, Recent Developments in Solar Photochemistry: Status and Perspectives, in: M. Becker, M. Böhmer (eds.) Proc. 8^{th} Int. Symp. Sol. Concentrating Technol., Oct. 6-11, 1996, Köln, Germany, C.F. Müller Verlag Heidelberg, (1997) Vol. 3 1325-1336; K.-H. Funken, J. Ortner, Technologies for the Solar Photochemical and Photocatalytic Manufacture of Specialities and Commodities: A Review, Z. Phys. Chem. 213 (1999) 99-105).

Seit langem ist die Verwendung voluminöser offener oder durch transparente Abdeckungen gegenüber der Umgebung abgeschirmter Reaktionsgefäße bekannt, die der Sonnenstrahlung ausgesetzt werden. In diesen Fällen werden die Eigenschaften des natürlichen Sonnenlichtes mit Ausnahme der durch Reflexionen an den Grenzflächen der Abdeckung und der durch Transmission durch die Abdeckung verursachten Abschwächung der spektralen Bestrahlungsstärke nicht verändert. Als eine jüngere Entwicklung auf diesem Gebiet wird beispielsweise in DE 198 44 037 A1 ein Flachbett-Sonnenlichtsammler/-Solarreaktor für solar-photo- und solar-thermochemische Synthesen beschrieben. Insbesondere bei hohen Konzentrationen bzw. bei großen Extinktionskoeffizienten eines homogen gelösten Chromophors sowie beim Einsatz suspendierter heterogener Katalysatorpartikel oder auch bei trüben Fluiden, wie beispielsweise Emulsionen, ist die Verwendung derartiger voluminöser Reaktionsgefäße jedoch nachteilig, denn die Eindringtiefe des Lichtes in die Reaktionsmischung ist infolge der Licht-Absorption entsprechend des Lambert-Beer'schen Gesetzes sowie infolge der Licht-Streuung im Fall der Katalysatorpartikel oder trüber Fluide sehr gering.

Die Verwendung dünner Schichten ist eine prinzipielle Lösung dieses Problems. Für die solare Detoxifizierung verunreinigter Wässer wurden daher Fallfilmreaktoren erprobt (D. Bahnemann, M. Meyer, U. Siemon, D. Mencke, A Self-Sufficient PV Powered Solar Detoxification Reactor for Polluted Waters, Proc. Int. Sol Energy Conf. Solar Engineering - 1997, April 27-30, 1997, ASME, Washington D.C., 261-267; B. Braun, J. Ortner, K.-H. Funken, M. Schäfer, C. Schmitz, G. Horneck, M. Fasdni, Dye-Sensitized Solar Detoxification and Disinfection of Contaminated Water, Proc. 8^{th} Int. Symp. Solar Thermal Concentrating Technologies, Vol. 3, C. F. Müller Verlag, Heidelberg (1997) 1391-1401). Es ist jedoch von Nachteil, dass bezogen auf das der Bestrahlung ausgesetzte Reaktionsvolumen großflächige Abdeckungen benötigt werden, die nur mit großem Aufwand zu fertigen sind, und eine beträchtliche Energie zum wiederholten Umpumpen der Reaktionsmischung über die Fallfilmfläche aufgewandt werden muss. Im Prinzip könnte man zwar auf die Abdeckung oberhalb des Fallfilms verzichten, dann würden aber ein Teil des Wassers sowie gegebenenfalls auch niedrigsiedende toxische Substanzen von der Fallfilmoberfläche verdampfen.

WO 95/06111 betrifft ein System unter Verwendung von röhrenförmigen Photobioreaktoren zur industriellen Züchtung von Mikroorganismen.

Der Bioreaktor ist im wesentlichen horizontal angeordnet und befindet sich zwischen zwei Kollektoren auf unterschiedlichem Niveau.

Eine ähnliche vergleichbare Anordnung wird in GB 2 118 572 A beschrieben.

Die FR 2 685 244 betrifft eine Vorrichtung ebenfalls zum Züchten von Mikroorganismen.

Die DE 197 46 343 A1 betrifft ein Verfahren und Vorrichtung zum Einbringen solarer Strahlungsenergien in einen Photoreaktor, bei dem auch im Inneren des Reaktors eine Reaktorröhre vorgesehen sein kann.

Jüngst wurden auch die nicht lichtkonzentrierenden Stegmehrfachplatten-Reaktoren, insbesondere Stegdoppelplatten-Reaktoren, die im wesentlichen aus einer oder mehreren flüssigkeitsdurchströmbaren Stegmehrfachplatten aus thermoplastisch extrudierbarem, transparentem oder transluzentem Kunststoff bestehen (EP 0 738 686 A1), und CPC-Reaktoren (Zusammengesetzte Parabol-Kollektoren) (beispielsweise J.I. Ajona, A. Vidal, The Use Of CPC Collectors For Detoxification Of Contaminated Water: Design, Construction And Preliminary Results, Solar Energy 68 (2000) 109-120) für die solare Detoxifzierung kontaminierter Wässer entwickelt und erprobt. Ein Reaktorenvergleich ergab, dass beide Reaktortypen durchaus interessante Alternativen für die solare Wasserreinigung sein können (R. Dillert, R. Goslich, J. Dzengel, H.-W. Schumacher, D. Bahnemann, Field Studies of Solar Water Detoxification, Proc. 1^{st} Users Workshop Training and Mobility of Researchers Programme at Plataforma Solar de Almería, Nov. 18-19, 1997, Almería, Spain, Ser. Ponencias, Madrid (1998) 31-40). Es wurde jedoch gefunden, dass die Stegdoppelplatten-Reaktoren in erheblichem Maß zum Fouling neigen und damit in ihrer Effiziens deutlich im Vergleich zu CPC-Reaktoren abfallen. Da die einzelnen Reaktionskanäle endständig verklebt sind, ist außerdem eine mechanische Reinigung nicht möglich. Hingegen wurde das Fouling bei den CPC-Reaktoren nicht beobachtet. Ein weiterer Nachteil der Stegmehrfachplatten-Reaktoren ist, dass bei einer Beschädigung einer Stegmehrfachplatte ein aus einem Bauteil bestehendes Reaktormodul komplett ausgetauscht werden muss. Ein derartiges Bauteil besitzt beispielsweise eine Fläche von 3,07 m². Bei den CPC-Kollektor-Reaktoren ist es von Nachteil, dass sie rückseitig eine kompliziert geformte Reflektorstruktur besitzen. Diese Reflektorstruktur stellt einen deutlichen Kostenfaktor dar. Je nach Qualität des Reflektormaterials muss im Langzeitbetrieb auch mit mechanischer und / oder optischer Degradation der Spiegelstrukturen gerechnet werden.

Es wurden auch linien- oder punktfokussierende Konzentratoren genutzt, um die Direktstrahlung der Sonne in den Reaktionsapparat zu bündeln, beispielsweise DE 43 44 163 A1. Bei den fokussierenden Konzentrator-Reaktoren ist die Bestrahlungsstärke größer als die des auf der Erdoberfläche auftreffenden natürlichen Sonnenlichtes. Dies kann für manche Anwendungen vorteilhaft sein. Es ist jedoch von Nachteil, dass die fokussierenden Apparate nur bei wolkenfreiem Himmel betrieben werden können. Daher ist ihr Betrieb zwar an guten Solar-Standorten attraktiv, allerdings ist ihre wirtschaftlich sinnvolle Verwendbarkeit bei weniger stabilen Wetterverhältnissen begrenzt. Außerdem sind Installation und Betrieb mit hohem Aufwand verbunden. Falls die höhere erreichbare Bestrahlungsstärke nicht prozessbedingt gefordert wird, ist der erhöhte technische und Kosten-Aufwand ausschlaggebend dafür, dass sie eher nicht eingesetzt werden.

Sowohl für photochemische als auch für photobiologische Anwendungen sind die Kosten zur Bereitstellung des Lichtes vön erheblicher Bedeutung. Bei elektrisch betriebenen Lichtquellen fallen vor allem hohe Investitionskosten für Lampen, Leistungsversorgung und Sicherheitsmaßnahmen sowie Betriebskosten für den Lampenersatz, der durch die begrenzte Lebensdauer der Lampen bedingt wird, sowie für Kühlung und für elektrischen Strom an.

Ausschließlich mit Sonnenlicht betriebene Systeme weisen nicht alle dieser Nachteile auf. Insbesondere entfallen die hohen Investitionskosten für Lampen und Leistungsversorgung sowie Betriebskosten für Lampenersatz und elektrischen Strom. Bei synthetischen Anwendungen ist der Aufwand für die Kühlung außerdem deutlich geringer als bei lampenbetriebenen Systemen. Von Nachteil gegenüber den lampenbetriebenen Systemen ist es aber vor allem, dass die solare Photochemie und Photobiologie nur während der Sonnenscheinstunden durchführbar ist. Allerdings besteht die Chance, dass an geeigneten Standorten mit Sonnenlicht betriebene photochemische oder photobiologische Systeme in einer Gesamtbetrachtung kostengünstiger arbeiten als mit Kunstlicht betriebene.

Der Erfindung liegt primär die Aufgabe zugrunde, eine Vorrichtung und ein Verfahren bereitzustellen, die die im Stande der Technik bekannten Probleme löst, die Kosten für den Solarreaktor minimiert, die Wartungsfreundlichkeit berücksichtigt und den Sonnenlicht-Lampenlicht-Hybridbetrieb gestattet.

Die Lösung der vorgenannten Aufgabe erfolgt erfindungsgemäß in einer ersten Ausführungsform durch eine Vorrichtung zur Durchführung solarer photochemischer oder photobiologischer Reaktionen, dadurch gekennzeichnet, dass der Photoreaktor aus einem oder mehreren transparenten äußeren Rohren besteht, die von außen durch Licht bestrahlt werden, wobei das Reaktionsmedium durch die Rohre geleitet und dabei der Strahlung ausgesetzt wird, wobei insbesondere konzentrisch in dem Inneren der äußeren Rohre ein oder mehrere leere transparente Rohre mit kleinerem Durchmesser angeordnet sind und das Reaktionsmedium durch den zwischen einem äußeren und einem inneren Rohr entstehenden Spalt oder im Falle mehrerer innerer Rohre durch die zwischen ihnen entstehenden Spalten geleitet wird.

Der Kern des erfindungsgemäßen Lösungsweges besteht darin, die Vorteile eines solaren Rohrreaktors zu nutzen, dabei aber auf komplizierte und kostenträchtige Bauteile zu verzichten. Zwar wird Sonnenlicht in einen CPC-Kollektor-Reaktor infolge der Reflexion des Sonnenlichtes an den rückwärtig angebrachten Parabolspiegeln effizienter eingetragen als in einen Rohrreaktor gemäß dieser Erfindung, aber dieser Nachteil wird durch eine vergrößerte Eingangsapertur bzw. Reaktionsdauer aufgewogen. Der Aufwand für Fertigung und Montage ist deutlich geringer als der für CPC-Kollektor-Reaktoren, denn die zusammengesetzten parabolischen Spiegel werden nicht benötigt. Außerdem kann das Problem der Spiegeldegradation nicht auftreten. Insgesamt ist der Mehraufwand für die Vergrößerung der Eingangsapertur für einen Photoreaktor entsprechend dieser Erfindung geringer als der Aufwand für die zusammengesetzten parabolischen Spiegel.

Im Vergleich zu Stegmehrfachplatten-Reaktoren können bei Bedarf mechanische Reinigungsarbeiten nach Lösen der Verbindungselemente zwischen zwei Rohrelementen einfach vorgenommen werden. Im Fall der Beschädigung eines Rohrelementes lässt sich dieses gezielt austauschen, was mit geringeren Kosten verbunden ist als der Austausch eines kompletten Reaktormoduls im Fall der Stegmehrfachplatten.

In einer Ausführungsform wird in dem Reaktionsrohr des Kollektors ein weiteres Rohr mit kleinerem Durchmesser gegebenenfalls konzentrisch angeordnet. Durch den Spalt zwischen diesen Röhren wird das zu bestrahlende Medium geleitet. Für jeweilige Reaktionssysteme kann die Spaltbreite mit den dem Fachmann bekannten Methoden leicht optimiert werden. Für einen nicht-konzentrierenden Solar-Kollektor-Reaktor kann man in dieser Ausführungsform ein hohes Verhältnis aus eingestrahlter Photonenmenge und Reaktionsvolumen ohne die Nachteile eines Fallfilm-Reaktors erzielen. In einer weiteren Ausführungsform werden mehrere Leerrohre in dem Reaktionsrohr angeordnet, wobei das Reaktionsmedium durch die Zwischenräume geleitet wird. Wenn man ein Rohr anordnet, kann man es konzentrisch anbringen, bei mehreren ist das nicht möglich. Es muss jedoch nicht notwendigerweise konzentrisch angeordnet sein. Bei mehreren Rohren wird das Reaktionsmedium auch nicht in einem Spalt zwischen 2 Rohren durchgeleitet, sondern in zwischen den Rohren entstehenden Spalten.

In einer weiteren besonderen Ausführungsform werden im Inneren des Solarreaktors eine oder mehrere Strahlungsquellen, insbesondere Lampen, beispielsweise Leuchtstoffröhren oder Mikrowellen-Plasma-Lampen in Verbindung mit Lichtleitern und Diffusoren, angeordnet, die eine hybride Arbeitsweise gestatten. Hierdurch wird der Betrieb der Vorrichtung vom Tag-Nacht-Zyklus und von meteorologischen Bedingungen unabhängig. Alternativ ist auch die Verwendung von Wärmequellen oder Kältequellen möglich, um eine photochemische oder photobiologische Reaktion bei einer gewünschten Temperatur durchzuführen. Bei ausreichendem Sonnenschein wird der Reaktor wie in der Grundversion durch das Außenrohr solar bestrahlt. Bei Bedarf liefern die Lampen die erforderliche Strahlung. Von Vorteil ist dabei, dass für den Lampenbetriebenen Anlagenteil keine gesonderte Investition für den Reaktorbehälter erforderlich ist, sondern lediglich Lampen beschafft und in das Innenrohr bzw. die Innenrohre positioniert werden müssen.

In einer alternativen Ausführungsform kann das von einer extern angeordneten Lampe gelieferte Licht auch durch lichtleitende Materialien in das Reaktorinnere geleitet werden. Diese Ausführungsform ist gegebenenfalls in Bezug auf die Lampe wartungsfreundlicher. Außerdem kann man bei dieser Ausführungsform auch alternativ zusätzliches Sonnenlicht in das Reaktorinnere leiten. Gegebenenfalls kann man auch nur einen Teil der Reaktionsrohre mit Lampen ausstatten. Bei vorhandener Sonneneinstrahlung kann man prozessangepasst den Eintrag an Sonnenstrahlung und an Lampenlicht miteinander mischen.

### Ausführungsbeispiele:

Zum Vergleich des erfindungsgemäßen Rohrreaktor, wurde exemplarisch der photokatalytische Abbau der biologisch schwer abbaubaren Modellsubstanzen Trichlorethylen (TCE, C₂HCl₃) und Ölsäuremethylester (OME, C₁₉H₃₆O₂) in wässriger Lösung bzw. Suspension untersucht. Bei den anderen Reaktortypen handelt es sich um einen Stegpdoppelplattenkollektor der Firma Solacryl, Elsenfeld, sowie einen CPC-Kollektor-Reaktor der Firma AO SOL, Portugal. Alle Reaktoren standen bei den Versuchen unverschattet nebeneinander an demselben Ort Köln-Porz. Sie waren alle in Ost-West-Richtung mit einer Elevation von 50° zur Sonne ausgerichtet. Alle Reaktoren besaßen außerdem die gleiche bestrahlte Fläche (3,07m²).

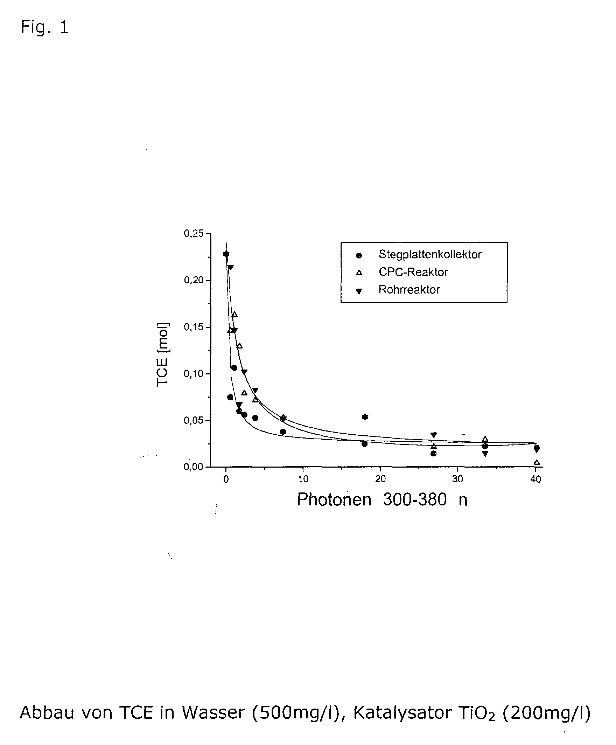

Fig. 1 zeigt, dass in allen drei Reaktortypen mit der gleichen Photonenmenge die gleiche Restkonzentration TCE erreicht werden kann.

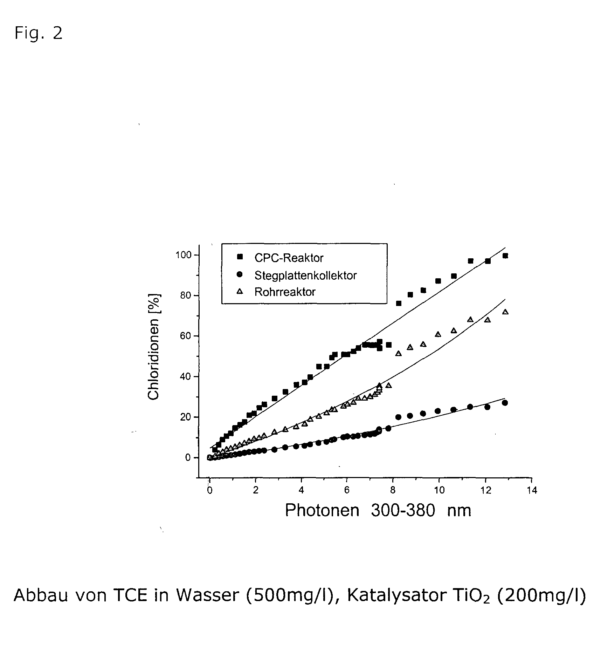

Fig. 2 zeigt, dass die Mineralisierung des TCE, hier beobachtet durch die Zunahme der Chlorid-Ionen-Konzentration, im CPC-Reaktor bei diesen Versuchen am effektivsten erfolgte. Wenn in ihm 100% der möglichen Chlorid-Ionen gebildet wurden, dann sind es im Rohrreaktor 72% und im Stegplattenkollektor lediglich 23%. Dabei ist jedoch zu beachten dass der konstruktive und materielle Aufwand zür Fertigung des CPC-Reaktor deutlich über dem des Rohreaktors liegt. Im Rohrreaktor und im CPC-Reaktor erfolgt die Chlorid-Ionen-Bildung sehr viel schneller als im Stegdoppelplattenkollektor.

Der Abbau von OME verläuft im Rohrreaktor sogar deutlich schneller und vollständiger als in den Vergleichsreaktoren.

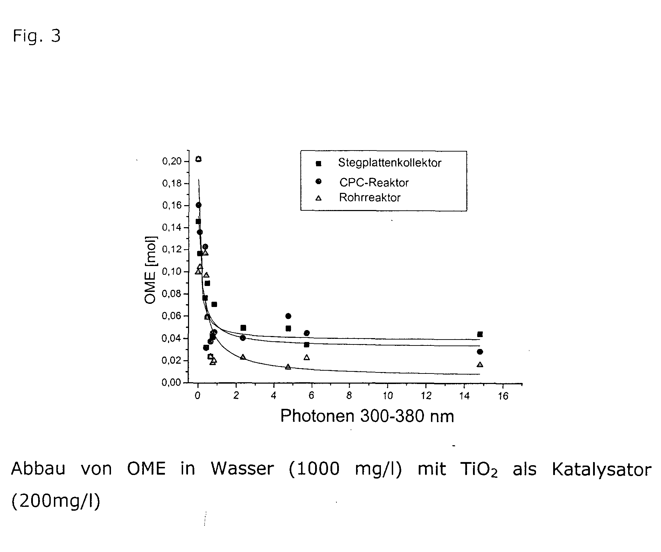

Aus Fig. 3 lässt sich ablesen, dass im Stegdoppelplattenkollektor und im CPC-Reaktor selbst nach einer Bestrahlung mit 15 mol Photonen jeweils ca. 80% des eingesetzten OME abgebaut werden. Im Rohrreaktor werden dagegen mit 2 mol Photonen bereits 90% des OME abgebaut und mit 10 mol Photonen mehr als 95%.

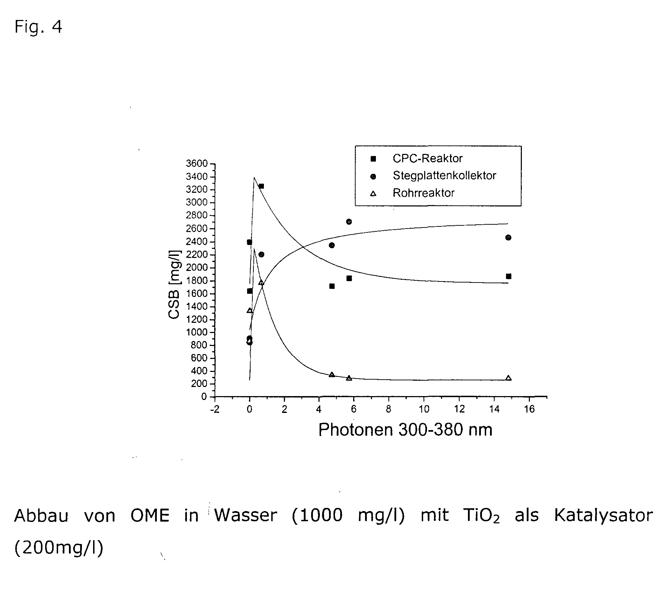

Dieses Ergebnis wird noch deutlicher, wenn man den chemischen Sauerstoffbedarf (CSB) als Messgröße in Fig. 4 betrachtet.

In allen drei Reaktoren wurde der gleiche Abbauversuch mit 1000mg/l OME durchgeführt. Im Stegplattenkollektor steigt der CSB vergleichsweise langsam an und bleibt auf einem hohen Niveau von ca. 70% des theoretischen Wertes konstant. Es findet kein weiterer Abbau der organischen Moleküle statt. Im CPC-Reaktor steigt der CSB sehr rasch auf einen Maximalwert und fällt dann entsprechend der Versuche mit TCE ab. Allerdings bleibt auch hier der Abbau bei einem CSB von 50% des theoretisch möglichen Wertes stehen. Im Rohrreaktor wird ein ähnlicher Abbau wie im CPC-Reaktor beobachtet, nur dass hier der Abbau nahezu vollständig ist.

## Patentansprüche

1. Vorrichtung zur Durchführung solarer photochemischer oder photobiologischer Reaktionen, dadurch gekennzeichnet, dass der Photoreaktor aus einem oder mehreren transparenten äußeren Rohren besteht, die von außen durch Licht bestrahlt werden, wobei das Reaktionsmedium durch die Rohre geleitet und dabei der Strahlung ausgesetzt wird, wobei insbesondere konzentrisch in dem Inneren der äußeren Rohre ein oder mehrere leere transparente Rohre mit kleinerem Durchmesser angeordnet sind und das Reaktionsmedium durch den zwischen einem äußeren und einem inneren Rohr entstehenden Spalt oder im Falle mehrerer innerer Rohre durch die zwischen ihnen entstehenden Spalten geleitet wird.

2. Vorrichtung nach Anspruch 1, dadurch gekennzeichnet, dass die transparenten Rohre aus Glas oder Kunststoffmaterial bestehen.

3. Vorrichtung nach einem der vorangehenden Ansprüche dadurch gekennzeichnet, dass im Inneren der inneren Rohre eine oder mehrere Strahlungsquellen, insbesondere rohrförmige Strahlungsquellen vorhanden sind.

4. Vorrichtung nach einem der vorangehenden Ansprüche dadurch gekennzeichnet, dass im Inneren der inneren Rohre lichtleitende Materialien oder Vorrichtungen eingebracht werden, durch die zusätzliches Sonnenlicht und / oder Licht einer oder mehrerer externer künstlicher Strahlungsquellen in das Reaktorinnere eingetragen wird.

5. Vorrichtung nach Anspruch 4, dadurch gekennzeichnet, dass die Strahlungsquellen Mikrowellen-Plasma-Lampen in Verbindung mit Lichtleitern und Diffusoren umfassen.

6. Vorrichtung nach einem oder mehreren der vorangehenden Ansprüche dadurch gekennzeichnet, dass die Reaktionsmedien enthaltenden Rohre in Serie und/oder parallel durchströmt werden.

7. Verfahren zur Durchführung solarer photochemischer oder photobiologischer Reaktionen, dadurch gekennzeichnet, dass man die Reaktion in einem Photoreaktor aus einem oder mehreren transparenten äußeren Rohren durchführt, die von außen und/oder gleichzeitig oder wechselweise auch von innen durch Licht bestrahlt werden, wobei das Reaktionsmedium durch die Rohre geleitet und dabei der Strahlung ausgesetzt wird.

8. Verfahren nach Anspruch 7, dadurch gekennzeichnet, dass man die photochemische oder photobiologische Reaktion mit Sonnenlicht und/oder künstlicher Strahlung betreibt.

9. Verfahren nach Anspruch 7 oder 8, dadurch gekennzeichnet, dass man die Reaktanden in flüssiger oder gasförmiger Phase rein, homogen oder heterogen, gegebenenfalls mit einem Lösemittel oder Trägergas vermischt durch die Reaktionsmedien enthaltenden Rohre leitet.

10. Verfahren nach einem der Ansprüche 7 bis 9, dadurch gekennzeichnet, dass man den Photoreaktor zur photochemischen oder photobiologischen Speicherung der Sonnenenergie einsetzt.

11. Verfahren nach einem der Ansprüche 7 bis 9, dadurch gekennzeichnet, dass man den Photoreaktor zur photochemischen oder photobiologischen Herstellung von Chemikalien einsetzt.

12. Verfahren nach einem der Ansprüche 7 bis 9, dadurch gekennzeichnet, dass man den Photoreaktor zur photonischen Detoxifikation und/oder Desinfektion von Fluiden, insbesondere verunreinigtem Wasser einsetzt.

13. Verfahren nach einem der Ansprüche 7 bis 9, dadurch gekennzeichnet, dass man den Photoreaktor zur photonischen Detoxifikation und/oder Desinfektion von verunreinigten Gasströmen, insbesondere Luft einsetzt.
